# EUROPEAN PATENT APPLICATION

(11) **EP 3 808 840 A1**
(43) Date of publication of application: **21.04.2021**
(21) Application number: 19819110.8
(22) Date of filing: 07.06.2019
(51) Int. Cl.: C12N 9/18, A61K 47/64, A61P 27/02, A61K 38/00

(54) **PHARMACEUTICAL COMPOSITION COMPRISING FUSION PROTEIN OF CELL-PENETRATING PEPTIDE AND RPE65 FOR TREATMENT OF LEBER'S CONGENITAL AMAUROSIS**

(30) Priority: 14.06.2018 KR 20180068383
(71) Applicant: Avixgen Inc., Seoul 06591 (KR)
(72) Inventor: SHIN, Woo Ri, Incheon 22134 (KR); BAEK, Yi Yong, Goyang-Si Gyeonggi-do 10375 (KR); CHOI, Jun Sub, Yongin-Si Gyeonggi-do 17027 (KR); KOO, Hye Cheong, Gwangmyeong-Si Gyeonggi-do 14314 (KR)
(74) Representative: Nony
(86) International application number: PCT/KR2019/006849
(87) International publication number: WO 2019/240431

(57) **Abstract**

The present disclosure relates to a composition including a conjugate of a cell-penetrating peptide and RPE65 (Retinal Pigment Epithelium-specific 65 kDa) as an active ingredient. The composition may be useful for the treatment of Leber's congenital amaurosis because RPE65 has increased cell permeability.

<110>    Avixgen Incorporation

<120>    Pharmaceutical Composition for Treatment of Leber's congenital
         amaurosis comprising Cell Penetrating Peptide and Retinal Pigment
         Epithelium-specific 65 kDa protein Conjugate

<130>    PN180167

<160>    6

<170>    KoPatentIn 3.0

<210>    1
<211>    56
<212>    PRT
<213>    Artificial Sequence

<220>
<223>    ACP amino acids


<400>    1
Met Gly Gln Arg Gly Asn Phe Arg Asn Gln Arg Lys Ile Val Lys Cys
  1               5                  10                  15

Phe Asn Cys Gly Lys Glu Gly His Thr Ala Lys Asn Cys Arg Ala Pro
             20                  25                  30

Arg Lys Lys Gly Cys Trp Lys Cys Gly Arg Glu Gly His Gln Met Lys
         35                  40                  45

Asp Cys Thr Glu Arg Gln Ala Asn
     50                  55


<210>    2
<211>    533
<212>    PRT
<213>    Artificial Sequence

<220>
<223>    RPE65 amino acids


<400>    2


<210>    3
<211>    589
<212>    PRT
<213>    Artificial Sequence

<220>
<223>    ACP-RPE65 amino acids


<400>    3
Met Gly Gln Arg Gly Asn Phe Arg Asn Gln Arg Lys Ile Val Lys Cys
 1               5                  10                  15

Phe Asn Cys Gly Lys Glu Gly His Thr Ala Arg Asn Cys Arg Ala Pro
            20                  25                  30

Arg Lys Lys Gly Cys Trp Lys Cys Gly Lys Glu Gly His Gln Met Lys
        35                  40                  45

Asp Cys Thr Glu Arg Gln Ala Asn Met Ser Ile Gln Val Glu His Pro
    50                  55                  60

Ala Gly Gly Tyr Lys Lys Leu Phe Glu Thr Val Glu Glu Leu Ser Ser
65                  70                  75                  80

Pro Leu Thr Ala His Val Thr Gly Arg Ile Pro Leu Trp Leu Thr Gly
                85                  90                  95

Ser Leu Leu Arg Cys Gly Pro Gly Leu Phe Glu Val Gly Ser Glu Pro
            100                 105                 110

Phe Tyr His Leu Phe Asp Gly Gln Ala Leu Leu His Lys Phe Asp Phe
        115                 120                 125

Lys Glu Gly His Val Thr Tyr His Arg Arg Phe Ile Arg Thr Asp Ala
    130                 135                 140

Tyr Val Arg Ala Met Thr Glu Lys Arg Ile Val Ile Thr Glu Phe Gly
145                 150                 155                 160

Thr Cys Ala Phe Pro Asp Pro Cys Lys Asn Ile Phe Ser Arg Phe Phe

165                 170                 175

Ser Tyr Phe Arg Gly Val Glu Val Thr Asp Asn Ala Leu Val Asn Val
        180                 185                 190

Tyr Pro Val Gly Glu Asp Tyr Tyr Ala Cys Thr Glu Thr Asn Phe Ile
    195                 200                 205

Thr Lys Ile Asn Pro Glu Thr Leu Glu Thr Ile Lys Gln Val Asp Leu
    210                 215                 220

Cys Asn Tyr Val Ser Val Asn Gly Ala Thr Ala His Pro His Ile Glu
225                 230                 235                 240

Asn Asp Gly Thr Val Tyr Asn Ile Gly Asn Cys Phe Gly Lys Asn Phe
                245                 250                 255

Ser Ile Ala Tyr Asn Ile Val Lys Ile Pro Pro Leu Gln Ala Asp Lys
            260                 265                 270

Glu Asp Pro Ile Ser Lys Ser Glu Ile Val Val Gln Phe Pro Cys Ser
        275                 280                 285

Asp Arg Phe Lys Pro Ser Tyr Val His Ser Phe Gly Leu Thr Pro Asn
    290                 295                 300

Tyr Ile Val Phe Val Glu Thr Pro Val Lys Ile Asn Leu Phe Lys Phe
305                 310                 315                 320

Leu Ser Ser Trp Ser Leu Trp Gly Ala Asn Tyr Met Asp Cys Phe Glu
                325                 330                 335

Ser Asn Glu Thr Met Gly Val Trp Leu His Ile Ala Asp Lys Lys Arg
            340                 345                 350

Lys Lys Tyr Leu Asn Asn Lys Tyr Arg Thr Ser Pro Phe Asn Leu Phe
        355                 360                 365

His His Ile Asn Thr Tyr Glu Asp Asn Gly Phe Leu Ile Val Asp Leu
    370                 375                 380

Cys Cys Trp Lys Gly Phe Glu Phe Val Tyr Asn Tyr Leu Tyr Leu Ala
385                 390                 395                 400

Asn Leu Arg Glu Asn Trp Glu Glu Val Lys Lys Asn Ala Arg Lys Ala
                405                 410                 415

Pro Gln Pro Glu Val Arg Arg Tyr Val Leu Pro Leu Asn Ile Asp Lys
            420                 425                 430

Ala Asp Thr Gly Lys Asn Leu Val Thr Leu Pro Asn Thr Thr Ala Thr
        435                 440                 445

Ala Ile Leu Cys Ser Asp Glu Thr Ile Trp Leu Glu Pro Glu Val Leu
    450                 455                 460

Phe Ser Gly Pro Arg Gln Ala Phe Glu Phe Pro Gln Ile Asn Tyr Gln
465                 470                 475                 480

Lys Tyr Cys Gly Lys Pro Tyr Thr Tyr Ala Tyr Gly Leu Gly Leu Asn
                485                 490                 495

His Phe Val Pro Asp Arg Leu Cys Lys Leu Asn Val Lys Thr Lys Glu

500                 505                 510

Thr Trp Val Trp Gln Glu Pro Asp Ser Tyr Pro Ser Glu Pro Ile Phe
        515                 520                 525

Val Ser His Pro Asp Ala Leu Glu Glu Asp Asp Gly Val Val Leu Ser
    530                 535                 540

Val Val Val Ser Pro Gly Ala Gly Gln Lys Pro Ala Tyr Leu Leu Ile
545                 550                 555                 560

Leu Asn Ala Lys Asp Leu Ser Glu Val Ala Arg Ala Glu Val Glu Ile
                565                 570                 575

Asn Ile Pro Val Thr Phe His Gly Leu Phe Lys Lys Ser
            580                 585

<210>    4
<211>    165
<212>    DNA
<213>    Artificial Sequence

<220>
<223>    ACP polynucleotides

<400>    4
cagaggggaa acttcagaaa ccagaggaag accgtgaagt gcttcaactg tggcaaggag      60

ggccacatcg ccaagaattg cagggcacca cgcaagaagg gatgctggag atgtggcaga     120

gagggacacc agatgaagga ctgtaccgag agacaggcca acctg                     165

<210>    5
<211>    1596
<212>    DNA
<213>    Artificial Sequence

<220>
<223>    RPE65 polynucleotides

<400>    5
agcatccaga tcgagcaccc agcaggaggc tacaagaagc tgtttgagac agtggaggag      60

ctgagctccc ctctgaccgc acacgtgaca ggacgcatcc cactgtggct gacaggcagc     120

ctgctgagat gcggccccgg cctgttcgaa gtgggctccg agcctttcta tcacctgttt     180

gacggccagg ccctgctgca caagttcgac ttcaaggagg gccacgtgac ataccaccgg     240

cggttcatca ggaccgacgc ctatgtgcgc gccatgacag agaagcggat cgtgatcacc     300

gagttcggca catgcgcctt tccagatccc tgtaagaata tcttctctag gttctttagc     360

tactttaagg gcgtggaggt gaccgacaac gccctggtga atatctatcc cgtgggcgag     420

gattactatg cctgcaccga gacaaacttc atcacaaaga tcaatcctga gacactggag     480

acaatcaagc aggtggacct gtgcaactac atctccgtga atggcgccac cgcccaccct     540

cacatcgagt ctgatggcac agtgtacaac atcggcaatt gcttcggcaa gaactttacc 600

gtggcctata atatcatcaa gatcccccct ctgaaggccg acaaggagga ccccatcaac 660

aagtctgagg tggtggtgca gttcccttgt agcgatcgct ttaagccatc ctacgtgcac 720

tctttcggcc tgaccccaaa ctatatcgtg tttgtggaga cacccgtgaa gatcaatctg 780

ttcaagtttc tgtctagctg gagcctgtgg ggcgccaact acatggactg cttcgagtcc 840

aatgagtcta tgggcgtgtg gctgcacgtg gccgataaga agaggcgcaa gtactttaac 900

aataagtatc ggacctcccc cttcaatctg tttcaccaca tcaacaccta cgaggacaat 960

ggcttcctga tcgtggatct gtgctgttgg aagggcttcg agttcgtgta caactatctg 1020

tacctggcca acctgagaga gaattgggag gaggtgaaga ggaatgccat gaaggcccct 1080

cagccagagg tgcggagata cgtgctgcct ctgaccatcg acaaggtgga tacaggcaga 1140

aacctggtga ccctgccaca caccacagcc accgccacac tgaggtctga cgagacaatc 1200

tggctggagc cagaggtgct gttcagcgga cctcggcagg ccttcgagtt tccacagatc 1260

aattaccaga agttcggcgg caagccatat acctacgcct atggcctggg cctgaaccac 1320

tttgtgcccg ataagctgtg caagctgaat gtgaagacaa aggagatctg gatgtggcag 1380

gagccagaca gctacccatc cgagcctatc ttcgtgagcc agcctgatgc cctggaggag 1440

gacgatggcg tggtgctgag cgtggtggtg tccccaggcg caggacagaa gccagcatat 1500

ctgctggtgc tgaacgccaa ggacctgtct gagatcgcca gagcagaagt ggagaccaat 1560

atccctgtca cctttcacgg gctgttcaag aggagc 1596

<210> 6
<211> 1761
<212> DNA
<213> Artificial Sequence

<220>
<223> ACP-RPE65 polynucleotides

<400> 6
cagaggggaa acttcagaaa ccagaggaag accgtgaagt gcttcaactg tggcaaggag 60

ggccacatcg ccaagaattg cagggcacca cgcaagaagg gatgctggag atgtggcaga 120

gagggacacc agatgaagga ctgtaccgag agacaggcca acctgagcat ccagatcgag 180

cacccagcag gaggctacaa gaagctgttt gagacagtgg aggagctgag ctcccctctg 240

accgcacacg tgacaggacg catcccactg tggctgacag gcagcctgct gagatgcggc 300

cccggcctgt tcgaagtggg ctccgagcct ttctatcacc tgtttgacgg ccaggccctg 360

ctgcacaagt tcgacttcaa ggagggccac gtgacatacc accggcggtt catcaggacc 420

gacgcctatg tgcgcgccat gacagagaag cggatcgtga tcaccgagtt cggcacatgc 480

gcctttccag atccctgtaa gaatatcttc tctaggttct ttagctactt taagggcgtg 540

gaggtgaccg acaacgccct ggtgaatatc tatcccgtgg gcgaggatta ctatgcctgc 600

accgagacaa acttcatcac aaagatcaat cctgagacac tggagacaat caagcaggtg 660

gacctgtgca actacatctc cgtgaatggc gccaccgccc accctcacat cgagtctgat 720

ggcacagtgt acaacatcgg caattgcttc ggcaagaact ttaccgtggc ctataatatc 780

atcaagatcc cccctctgaa ggccgacaag gaggacccca tcaacaagtc tgaggtggtg 840

gtgcagttcc cttgtagcga tcgctttaag ccatcctacg tgcactcttt cggcctgacc 900

ccaaactata tcgtgtttgt ggagacaccc gtgaagatca atctgttcaa gtttctgtct 960

agctggagcc tgtggggcgc caactacatg gactgcttcg agtccaatga gtctatgggc 1020

gtgtggctgc acgtggccga taagaagagg cgcaagtact ttaacaataa gtatcggacc 1080

tcccccttca atctgtttca ccacatcaac acctacgagg acaatggctt cctgatcgtg 1140

gatctgtgct gttggaaggg cttcgagttc gtgtacaact atctgtacct ggccaacctg 1200

agagagaatt gggaggaggt gaagaggaat gccatgaagg cccctcagcc agaggtgcgg 1260

agatacgtgc tgcctctgac catcgacaag gtggatacag gcagaaacct ggtgaccctg 1320

ccacacacca cagccaccgc cacactgagg tctgacgaga caatctggct ggagccagag 1380

gtgctgttca gcggacctcg gcaggccttc gagtttccac agatcaatta ccagaagttc 1440

ggcggcaagc catataccta cgcctatggc ctgggcctga accactttgt gcccgataag 1500

ctgtgcaagc tgaatgtgaa gacaaaggag atctggatgt ggcaggagcc agacagctac 1560

ccatccgagc ctatcttcgt gagccagcct gatgccctgg aggaggacga tggcgtggtg 1620

ctgagcgtgg tggtgtcccc aggcgcagga cagaagccag catatctgct ggtgctgaac 1680

gccaaggacc tgtctgagat cgccagagca gaagtggaga ccaatatccc tgtcaccttt 1740

cacgggctgt tcaagaggag c 1761

## Description

### Technical Field

The present disclosure relates to a conjugate of a cell-penetrating peptide and RPE65 and a pharmaceutical composition for treating Leber's congenital amaurosis including the same as an active ingredient.

### Background Art

Amaurosis refers to visual disturbances that cannot recognize an object although having no particular abnormalities when viewed externally, and is also called black cataract. Leber's congenital amaurosis (LCA), a type of amaurosis, is a hereditary retinal disease that causes congenital blindness, and is characterized by severe vision loss, strabismus, eye tremor, glare, and retinitis pigmentosa.

As gene mutations that cause Leber's congenital amaurosis, about 17 mutations, including RPE65 (retinal pigment epithelium-specific 65 kDa), CEP290 (centrosomal protein 290 kDa), GUCY2D (retinal guanylate cylase-1), CRB1 (crumbs homolog 1), AIPL1 (aryl hydrocarbon interacting protein like 1), LCA5, and CRX (cone rod homeobox), have been known to date, which are all mutations associated with photoreceptor cells and retinal pigment epithelium cells.

RPE65 is a 65-kDa retinoid isomerase present in the retinal pigment epithelium, and synthesizes 11-cis-retinol that acts as a chromophore in photoreceptor cells. Leber's congenital amaurosis caused by the RPE65 mutation accounts for 10% of Leber's congenital amaurosis cases.

The present inventors have conducted studies on a method for delivering a normal RPE65 protein into the retinal pigment epithelium cells of a Leber's congenital amaurosis patient, and have found that the use of a cell-penetrating peptide derived from the HIV (human immunodeficiency virus nucleocapsid can increase the cell permeability of RPE65, thereby completing the present disclosure.

### DISCLOSURE

### Technical Problem

It is an object of the present disclosure to provide a conjugate including a cell-penetrating peptide and RPE65 (Retinal Pigment Epithelium-specific 65 kDa protein), a pharmaceutical composition for treating Leber's congenital amaurosis including the same as an active ingredient, a method for treating Leber's congenital amaurosis, and the use thereof.

### Technical Solution

One aspect of the present disclosure provides a conjugate including: a cell-penetrating peptide including the amino acid sequence of SEQ ID NO: 1; and RPE65 (Retinal Pigment Epithelium-specific 65 kDa protein) consisting of the amino acid sequence of SEQ ID NO: 2.

Another aspect of the present disclosure provides a polynucleotide encoding the conjugate.

Still another aspect of the present disclosure provides a recombinant vector including the polynucleotide.

Yet another aspect of the present disclosure provides a host cell including the recombinant vector.

Still yet another aspect of the present disclosure provides a method for producing a conjugate, the method including steps of: (a) culturing the host cell in a culture medium; and (b) recovering the conjugate from the culture medium.

A further aspect of the present disclosure provides a pharmaceutical composition for treating Leber's congenital amaurosis including the conjugate as an active ingredient.

Another further aspect of the present disclosure provides a method for treating Leber's congenital amaurosis including a step of administering the composition to a subject.

Still another further aspect of the present disclosure provides the use of the conjugate in the manufacture of a medicament for treating Leber's congenital amaurosis.

### Advantageous Effects

The conjugate of the cell-penetrating peptide and RPE65 has increased cell permeability, and thus may be useful for the treatment of Leber's congenital amaurosis.

### Brief Description of Drawings

FIG. 1 shows the results of analyzing the cell membrane-penetrating activity of an ACP-RPE65M conjugate in HeLa cells.
FIG. 2 shows the results of analyzing the enzymatic activity of the ACP-RPE65M conjugate by HPLC (high-performance liquid chromatography).
FIG. 3 shows the results of analyzing the penetration activity of an ACP-conjugated RPE65 protein into retinal pigment epithelium cells.

### Best Mode

To achieve the above object, one aspect of the present disclosure provides a conjugate including: a cell-penetrating peptide including the amino acid sequence of SEQ ID NO: 1; and RPE65 (Retinal Pigment Epithelium-specific 65 kDa protein) consisting of the amino acid sequence of SEQ ID NO: 2.

As used herein, the term "cell-penetrating peptide (CPP)" refers to a cell membrane-permeable peptide consisting of a short peptide of about 10 to about 60 amino acids, which can move into a cell without damaging the cell membrane and can intracellularly deliver even a DNA or protein that cannot pass through the cell membrane.

As used herein, the term "RPE65 (Retinal Pigment Epithelium-specific 65 kDa)" refers to an enzyme that synthesizes 11-cis-retinol in retinal epithelium cells. If mutation in the RPE65 gene occurs, the conversion of trans-retinol to cis-retinol cannot occur, and thus the optic nerve cannot function normally.

As used herein, the term "conjugate" refers to a substance in which a cell-penetrating peptide and a biologically or pharmaceutically active protein are linked together by a chemical/physical covalent or non-covalent bond.

In one embodiment of the present disclosure, the expression "biologically or pharmaceutically active protein" capable of forming the conjugate by binding to the cell-penetrating peptide means a protein that can regulate physiological phenomena *in vivo.* The expression includes proteins, peptides, lipids linked to proteins or peptides, carbohydrates, chemical compounds, or fluorescent labels. For example, RPE65 having the amino acid sequence of SEQ ID NO: 2 may be used as the protein. The cell-penetrating peptide may be linked to the N-terminus of RPE65 to form the conjugate.

In one embodiment of the present disclosure, the cell-penetrating peptide including the amino acid sequence of SEQ ID NO: 1 may be encoded by the polynucleotide sequence of SEQ ID NO: 4, and the RPE65 protein set forth in the amino acid sequence of SEQ ID NO: 2 may be encoded by the polynucleotide sequence of SEQ ID NO: 5.

In one embodiment of the present disclosure, the conjugate may include the amino acid sequence of SEQ ID NO: 3 (ACP-RPE65).

Another aspect of the present disclosure provides a polynucleotide encoding the conjugate.

As used herein, the term "polynucleotide" refers to a polymer of deoxyribonucleotide or ribonucleotide that exists in a single-stranded or double-stranded form. The term includes RNA genome sequences, DNA (gDNA and cDNA), and RNA sequences transcribed therefrom, and includes analogues of natural polynucleotide, unless otherwise indicated.

In one embodiment of the present disclosure, the polynucleotide includes not only a nucleotide sequence encoding the conjugate, but also a sequence complementary thereto. The complementary sequences include not only completely complementary sequences, but also substantially complementary sequences. In addition, the polynucleotide sequence may be modified, and such modifications include addition, deletion or non-conservative substitution or conservative substitution of nucleotides.

In one embodiment of the present disclosure, the polynucleotide encoding the conjugate may include the polynucleotide sequence set forth in SEQ ID NO: 6 (ACP-RPE65).

Still another aspect of the present disclosure provides a recombinant vector including the polynucleotide encoding the conjugate.

As used herein, the term "vector" refers to a means for expressing a target gene in a host cell. Examples of the vector include, but are not limited to, plasmid vectors, cosmid vectors, and viral vectors such as bacteriophage vectors, adenoviral vectors, retroviral vectors and adeno-associated viral vectors. Vectors that may be used as the recombinant vector may be constructed by engineering plasmids (e.g., pSC101, pGV1106, pACYC177, ColE1, pKT230, pME290, pBR322, pUC8/9, pUC6, pBD9, pHC79, pIJ61, pLAFR1, pHV14, pGEX series, pET series, and pUC19, etc.), phages (e.g., λgt4λB, λ-Charon, λΔz1, and M13, etc.), or viruses (e.g., CMV, SV40, etc.), which are generally in the art.

The recombinant vector may include the polynucleotide sequence and a promoter operatively linked to the polynucleotide sequence.

As used herein, the term "operably linked" refers to a functional linkage between a nucleotide expression control sequence (e.g., a promoter sequence) and another nucleotide sequence, whereby the control sequence controls the transcription and/or translation of the other nucleotide sequence.

Recombinant vectors that may be used in the present disclosure may be constructed by engineering plasmids (e.g., pSC101, ColE1, pBR322, pUC8/9, pHC79, pUC19, pET, etc.), phages (e.g., λgt4λB, λ-Charon, λΔz1 and M13, etc.), or viruses (e.g., SV40, etc.), which are generally used in the art.

The recombinant vector may include a tag sequence facilitating purification of the conjugate of the cell-penetrating peptide and RPE65, for example, a continuous histidine codon, a maltose-binding protein codon, a Myc codon, etc. and may further include a fusion partner for increasing the solubility of the conjugate. In addition, the recombinant vector may include a sequence that is specifically cleaved by an enzyme in order to remove an unnecessary portion when the conjugate is expressed, an expression control sequence, and a marker or reporter gene sequence for identifying intracellular delivery.

Yet another aspect of the present disclosure provides a host cell including the recombinant vector, that is, a cell transformed with the recombinant vector.

A host cell that is capable of stably and consecutively cloning or expressing the recombinant vector may be any host cell publicly-known in the art. Examples of prokaryotic cells include *E. coli* JM109, *E. coli* BL21, *E*. *coli* RR1, *E. coli* LE392, *E. coli* B, *E. coli* X 1776, and *E*. *coli* W3110. When the recombinant vector is transformed into eukaryotic cells, *Saccharomyce cerevisiae,* insect cells, plant cells and animal cells, for example, SP2/0, CHO (Chinese hamster ovary) K1, CHO DG44, PER.C6, W138, BHK, COS-7, 293, HepG2, Huh7, 3T3, RIN and MDCK cell lines, may be used as host cells.

The polynucleotide or the recombinant vector including the same may be transferred into the host cell using a transfer method widely known in the art. As the transfer method, for example, when the host cell is a prokaryotic cell, a CaCl₂ method or an electroporation method may be used, and when the host cell is a eukaryotic cell, a microinjection method, a calcium phosphate precipitation method, an electroporation method, a liposome-mediated transfection method or a gene bombardment method may be used, but the transfer method is not limited thereto.

A method of selecting the transformed host cell may be easily carried out using a phenotype expressed by a selection marker according to a method known in the art. For example, when the selection marker is a specific antibiotic resistance gene, a transformant may be easily selected by culturing the transformant in a medium containing the antibiotic.

Still yet another aspect of the present disclosure provides a method for producing the conjugate of the cell-penetrating peptide and RPE65, the method including steps of: culturing the host cell in a culture medium; and recovering the conjugate from the culture medium.

In one embodiment of the present disclosure, the culture of the cell may be large-scale cell culture, and the culture of the cell may be performed using a cell culture method which is commonly used. For example, the cell culture method may be any one selected from the group consisting of batch culture, repeated batch culture, fed-batch culture, repeated fed-batch culture, continuous culture, and perfusion culture, but is not limited thereto.

In one embodiment of the present disclosure, the step of recovering the conjugate from the culture medium may be performed using various separation and purification methods publicly-known in the art. Generally, the cell lysate may be centrifuged to remove cell debris, culture impurities, etc., and then precipitation, for example, salting out (ammonium sulfate precipitation and sodium phosphate precipitation), solvent precipitation (protein fraction precipitation using acetone, ethanol, isopropyl alcohol, etc.) or the like, may be performed, and dialysis, electrophoresis, and various column chromatographies may be performed.

A further aspect of the present disclosure provides a pharmaceutical composition for treating Leber's congenital amaurosis including the conjugate as an active ingredient.

As used herein, the term "Leber's congenital amaurosis" refers to a hereditary retinal disease that causes congenital blindness. Leber's congenital amaurosis is caused by gene mutations such as RPE65 (retinal pigment epithelium-specific 65 kDa), CEP290 (centrosomal protein 290 kDa), GUCY2D (retinal guanylate cylase-1), CRB1 (crumbs homolog 1), and AIPL1 (aryl hydrocarbon interacting protein like 1).

The pharmaceutical composition of the present disclosure may include a pharmaceutically acceptable carrier, if necessary, in addition to the conjugate.

These pharmaceutically acceptable carriers are those that are commonly used in the manufacture of pharmaceuticals, and examples thereof include, but are not limited to, lactose, dextrose, sucrose, sorbitol, mannitol, starch, gum acacia, calcium phosphate, alginate, gelatin, calcium silicate, microcrystalline cellulose, polyvinylpyrrolidone, cellulose, water, syrup, methyl cellulose, methylhydroxybenzoate, propylhydroxybenzoate, talc, magnesium stearate, mineral oil, and the like. In addition, the pharmaceutical composition of the present disclosure may further include additives such as a lubricant, a wetting agent, a sweetener, a flavoring agent, an emulsifying agent, a suspending agent, a preservative, etc.

The carrier may be included in an amount of about 1 wt% to about 99.99 wt%, preferably about 90 wt% to about 99.99 wt%, based on the total weight of the pharmaceutical composition of the present disclosure, and the additives may be included in an amount of about 0.1 wt% to about 20 wt% based on the total weight of the pharmaceutical composition of the present disclosure.

Meanwhile, the pharmaceutical composition of the present disclosure may be administered orally or parenterally, but may be administered directly to the skin by a topical administration method.

The pharmaceutical composition of the present disclosure may be formulated with a pharmaceutically acceptable carrier and/or excipient and prepared in a unit dose form or contained in a multi-dose container. In this regard, the formulation may be in the form of a solution, a suspension or an emulsion, or may include elixir, extract, powder, granule, tablet, plaster, liniment, lotions, ointment, etc.

The daily dose of the pharmaceutical composition of the present disclosure may generally be in the range of 0.001 to 150 mg/kg of body weight, and may be administered once or several times. However, the dose of the pharmaceutical composition of the present disclosure is determined in view of various related factors such as the route of administration, the patient's age, sex and body weight, and the severity of the patient, and thus the dose should not be understood to limit the scope of the present disclosure in any way.

Another further aspect of the present disclosure provides a method for treating Leber's congenital amaurosis including a step of administering the composition to a subject.

The subject refers to an animal, and may typically be a mammal capable of exhibiting a beneficial effect by treatment with the conjugate of the present disclosure. Preferred examples of such subjects may include primates such as humans. In addition, such subjects may include all subjects who have symptoms of Leber's congenital amaurosis or are at risk of having such symptoms.

Still another further aspect of the present disclosure provides the use of the conjugate in the manufacture of a medicament for treating Leber's congenital amaurosis.

### Mode for Invention

Hereinafter, the present disclosure will be described in more detail with reference to one or more examples. However, these examples are to illustrate the present disclosure, and the scope of the present disclosure is not limited to these examples.

### Example 1: Preparation of Cell Penetrating Peptide-RPE65M Conjugate

As a cell-penetrating peptide, ACP (Avixgen's advanced cell penetrating peptide (hereinafter referred to as ACP); SEQ ID NO: 1) was used. In order to examine whether a conjugate of ACP and RPE65M is expressed and to purify the protein, a recombinant vector was constructed as follows.

To express the ACP-RPE65M protein, the ACP-RPE65M gene was inserted into a pET28a vector using restriction enzymes. The recombinant vector having the ACP-RPE65M gene inserted therein was named pET28a ACP-RPE65M-1.

To replicate the pET28a ACP-RPE65M-1 vector, the vector was transformed into *E. coli* DH5α which was then shake-cultured in LB liquid medium at 37°C until the OD reached 0.5 to 0.6. After completion of the culture, the cell culture was centrifuged, and the *E. coli* pellet was collected. The pET28a ACP-RPE65M-1 vector was isolated from the collected *E. coli* pellet using a plasmid extraction kit (Qiagen) according to the manufacturer's protocol. The isolated pET28a ACP-RPE65M-1 vector was quantified for its concentration using a UV-spectrophotometer, and then identified.

In addition, in order to express ACP-RPE65M in insect cells, baculovirus was constructed using a pFastBac1-MBP vector, and an ACP-RPE65M-2 recombinant bacmid which is expressed in insect cells was obtained using the above-described method of replicating the vector in *E. coli.*

### Example 1-2: Expression and Purification of ACP-RPE65M

In order to examine whether the pET28a ACP-RPE65M-1 vector and recombinant bacmid ACP-RPE65M-2 constructed as described above express protein, an experiment was performed as follows.

BL21(DE3) (Thermo Fisher, USA) was transformed with the pET28a ACP-RPE65M-1 vector, streaked on an LB plate, and then cultured at 37°C for 12 hours. After 12 hours, the formed colony was inoculated into an LB liquid medium and further cultured at 37°C. After about 12 hours, the cell culture that reached an OD of 0.5 to 0.6 was inoculated into 250 ml of an LB liquid medium and cultured at 37°C for 3 to 4 hours so as to reach an OD of 0.4 to 0.6. When the OD of the cell culture reached 0.4 to 0.6, 0.25 mM IPTG (isopropyl β-D-thiogalactoside) was added to the cell culture which was then cultured at 16°C for 18 hours. After 18 hours, the cell culture was centrifuged, and the BL21 pellet was collected and suspended in a lysis buffer (20 mM Tris, 300 mM NaCl, 5 mM imidazole and pH 8.0), and then the *E. coli* cells were lysed by sonication at an amplitude of 35%.

The *E. coli* cell lysate was separated into a supernatant and a pellet by centrifugation, and a tube was filled with the supernatant by using a 0.45 µm filter. The tube filled with the supernatant was placed in a column packed with Ni-NTA (nitrilotriacetic acid) resin, so that protein was bound to the resin. Thereafter, in order to remove foreign protein that did not bind to the resin, the resin was washed by adding a washing buffer (20 mM Tris, 300 mM NaCl, 30 mM imidazole and pH 8.0) thereto. Using a buffer (20 mM Tris, 300 mM NaCl, 400 mM imidazole and pH 8.0) containing imidazole, a final protein was obtained with the gradient mobile phase. Next, to remove imidazole, the obtained protein was placed in a membrane tube and subjected to buffer exchange by osmotic action using an imidazole-free buffer (20 mM NaH₂PO₄, 200 mM NaCl, 2 mM DTT, 10% glycerol and pH 7.0). Finally, the concentration of the protein dissolved in the imidazole-free buffer was measured by Bradford assay, thereby confirming expression of the ACP-RPE65M-1 protein. The ACP-RPE65M-2 recombinant bacmid was expressed in SF9 cells, and then the ACP-RPE65M-2 protein was obtained by separation and purification according to the above-described method.

### Example 2: Analysis of ACP-RPE65M Conjugate

### 2-1. Analysis of Cell Membrane Permeability of ACP-RPE65M

HeLa cells were seeded into a 12-well plate containing glass at a density of 1x10⁵ cells/well, and cultured for 24 hours so as to be attached to the glass. Thereafter, the HeLa cells were treated with 1 µM ACP-RPE65M, and after 3 hours, the cells were washed three times with PBS. The washed cells were fixed with 3.7% formaldehyde for 20 minutes, and treated with PBS containing 0.2% Triton X-100, thus increasing cell membrane permeability. Thereafter, the cells were blocked with 3% BSA for 1 hour, incubated with ACP antibody (Abcam, ab9108) at room temperature for 2 hours, and then washed three times with PBS. The cells were treated with Alexa 488 secondary antibody, incubated at room temperature for 1 hour, washed twice with PBS, and then stained with DAPI (4',6-diamidino-2-phenylindole) for 10 minutes. The glass having the HeLa cells attached thereto was detached, placed on slide glass, and observed by confocal laser scanning microscopy (LSM 700, Zeiss, Germany).

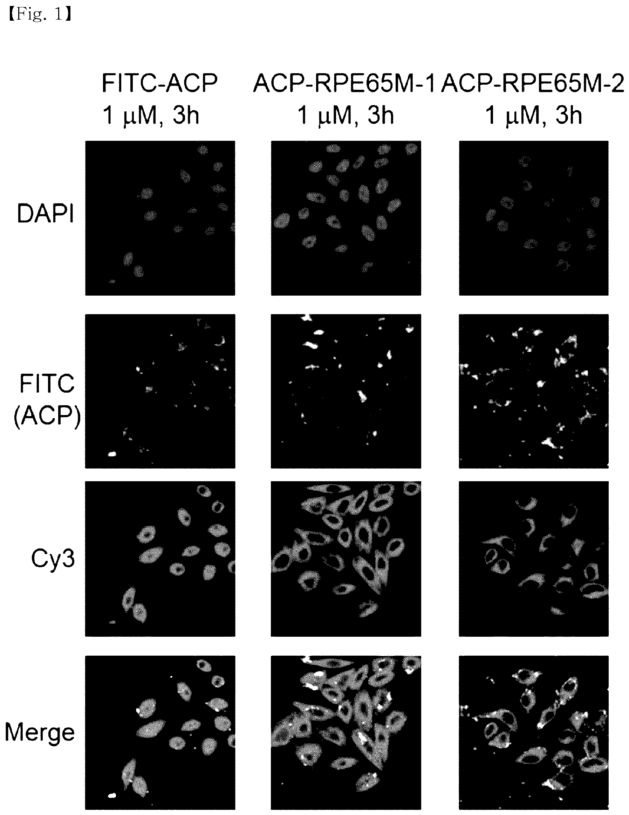

As a result, as shown in FIG. 1, the FITC fluorescence signal was observed within the cells, suggesting that ACP-RPE65M entered the HeLa cells through the cell membrane. In FIG. 1, ACP-RPE65M-1 is a conjugate expressed in and purified from *E. coli,* and ACP-RPE65M-2 is a conjugate expressed using baculovirus and purified.

### 2-2. Analysis of Enzymatic Activity of ACP-RPE65M

10 µg of the ACP-RPE65M conjugate and 200 µl of 4 mM retinyl acetate (in 10 mM Tris pH 7.4) as all-trans-retinol were mixed together, and then reacted at 37°C for 1 hour. The reaction was terminated by addition of 300 µl of methanol, and 300 µl of hexane was added to the reaction mixture which was then vortexed. Thereafter, the supernatant was collected by centrifugation, and then all-trans-retinol and 11-cis-retinol therein were analyzed by HPLC.

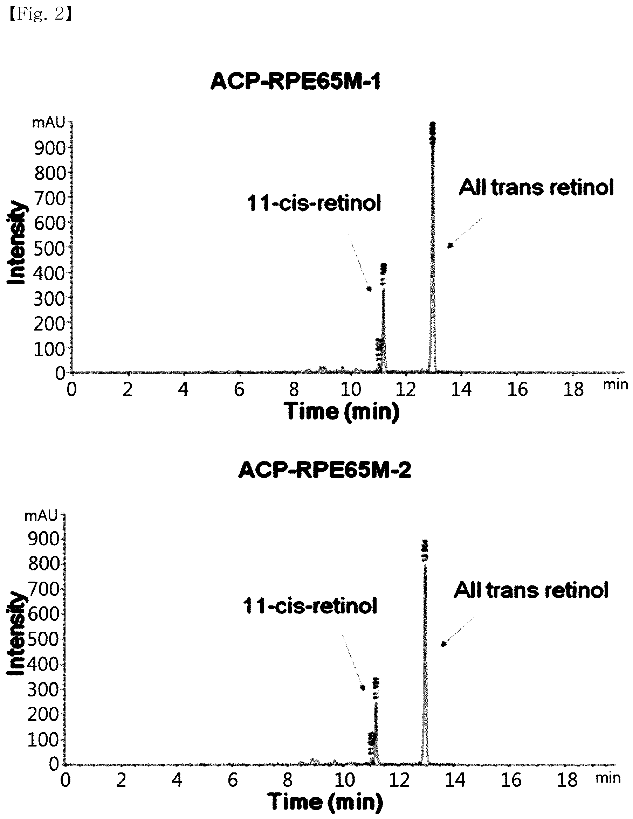

As a result, as shown in FIG. 2, it could be confirmed that 11-cis-retinol was produced by ACP-RPE65M. This suggests that the ACP-RPE65M conjugate retains the enzymatic activity of RPE65.

### Example 3: Analysis of Penetration of ACP-RPE65 Protein into Retinal Epithelium Cells

In order to examine whether the ACP-RPE65 protein is delivered into retinal pigment epithelium cells and actually exhibits the enzymatic activity of RPE65, an experiment was performed as follows.

Specifically, C57BL/6 mice were anesthetized with 2% avertin, and each of 1 µL of ACP-RPE65 containing 100 mM His-tag and 1 µL of RPE65 solution containing His-tag was injected subretinally. At 6 hours after injection, the mice were euthanized with excess anesthesia, and the eyeballs were enucleated, and then fixed in 4% paraformaldehyde at room temperature for 1 hour. After fixation, each eyeball was transferred into PBS and washed for 30 minutes, and then the cornea and lens were removed under a dissecting microscope. Next, eye cups were made and then transferred to a 24-well dish. Then, RPE65 was visualized by immunostaining using His-tag antibody. The eye cups were incubated with anti-His-tag for 2 hours, washed with phosphate buffered saline, and then immunostained with fluorescent probe-labeled secondary antibody. Each retina was spread out to be viewed from the top and was covered with cover glass to prepare slides. The prepared slides were observed under a fluorescence microscope and it was examined whether RPE65 was delivered into the retinal pigment epithelium cells, by immunostaining using anti-his-tag in the retinal epithelium cells of the eyeball into which each of ACP-RPE65 and RPE65 was injected subretinally.

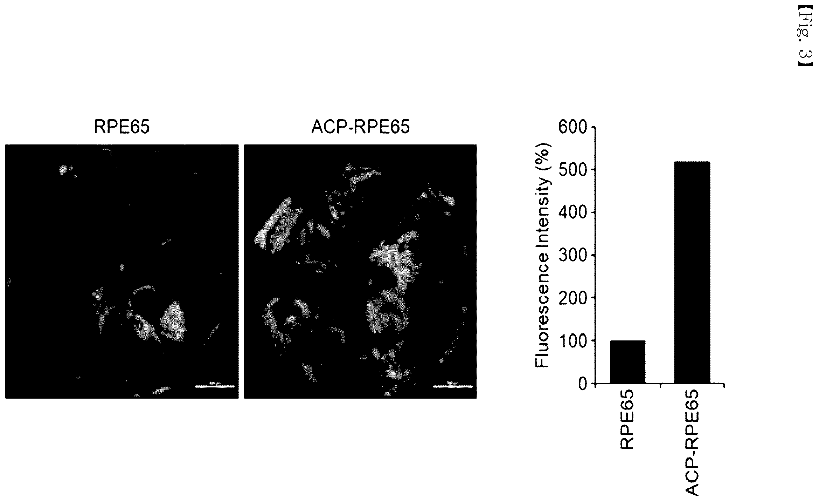

As a result, as can be seen in FIG. 3, it was confirmed that the ACP-RPE65 protein was delivered into the retinal pigment epithelium cells and that the delivery rate of RPE65, to which ACP was not conjugated, into the retinal pigment epithelium cells, was low.

These results suggest that ACP has the ability to deliver the RPE65 protein into retinal pigment epithelium cells.

The present disclosure has been described above with reference to the embodiments. Those skilled in the art to which the present invention pertains will appreciate that the present disclosure may be embodied in modified forms without departing from the essential characteristics of the present disclosure. Therefore, it should be understood that the disclosed embodiments are illustrative in all aspects and are not restrictive. The scope of the present disclosure is defined by the claims rather than the foregoing description, and all differences within the scope equivalent to the claims should be construed as falling within the scope of the present invention.

## Claims

1. A conjugate comprising:
a cell-penetrating peptide comprising the amino acid sequence of SEQ ID NO: 1; and
RPE65 (Retinal Pigment Epithelium-specific 65 kDa protein) consisting of the amino acid sequence of SEQ ID NO: 2.

2. A polynucleotide encoding the conjugate of claim 1.

3. A recombinant vector comprising the polynucleotide of claim 2.

4. A host cell comprising the recombinant vector of claim 3.

5. A method for producing a conjugate, the method comprising steps of:
(a) culturing the host cell of claim 4 in a culture medium; and
(b) recovering the conjugate from the culture medium.

6. A pharmaceutical composition for treating Leber's congenital amaurosis comprising the conjugate of claim 1 as an active ingredient.

7. A method for treating Leber's congenital amaurosis, the method comprising a step of administering the composition of claim 6 to a subject.

8. Use of the conjugate of claim 1 in the manufacture of a medicament for treating Leber's congenital amaurosis.
